Europäisches Patentamt

European Patent Office

Office européen des brevets

(19)

(11) Publication number: **0 137 528**
**A1**

(12) # EUROPEAN PATENT APPLICATION

(21) Application number: **84201057.1**

(22) Date of filing: **16.07.84**

(51) Int. Cl.⁴: **A 61 M 25/00**
**A 61 M 25/02, A 61 M 1/00**

(30) Priority: **26.07.83 NL 8302648**

(43) Date of publication of application:
**17.04.85 Bulletin 85/16**

(84) Designated Contracting States:
**AT BE CH DE FR GB IT LI LU NL SE**

(71) Applicant: **Fundatech S.A.**
**72, Rue du Grand-Pré**
**CH-1202 Geneva(CH)**

(72) Inventor: **Spits, Marc**
**Odilialaan 125**
**B-3590 Achel(BE)**

(74) Representative: **Kupecz, Arpad et al,**
**Octrooibureau Los en Stigter B.V. Postbox 20052**
**NL-1000 HB Amsterdam(NL)**

(54) **Device for supplying a fluid substance into and draining it from the maxillary antrum.**

(57) The present invention relates to a device for supplying a fluid substance into and draining it from the maxillary antrum. Said device comprises a flexible tube (1) as well as at least one arrester (4) connected to one of the ends of the tube (1). By the use of the device of the invention in the treatment of inflammations of the maxillary antrum the patient can be saved an appreciable part of the pain. Furthermore said device has the advantage that it can be manufactured at low cost because of its simplicity, and that it can be sterilized in a simple manner.

fig.2

0137528

Device for supplying a fluid substance into and draining it from the maxillary antrum.

In the presence of conditions, specifically inflammations, of the maxillary antrum (antrum highmori, maxillary sinus), possibilities are available for internal examination of the state of the maxillary antrum with the aid of a specially adapted endoscope, also known as an antroscope. To this end, access is provided through the nose to the maxillary antrum, which is an air cavity bounded by mucous membrane and opening into the central nasal duct, use being made of a so-called antroscopic troicart. The probe of the antroscope can be introduced into the maxillary antrum through the cannula of the antroscopic troicart.

A liquid inflammation product (pus) is sometimes encountered in the maxillary antrum. Discharge of fluid from the maxillary antrum to the nose is usually possible through an outlet passage which opens below the central turbinal, and therefore into the central nasal duct. In case of the maxillary antrum being inflamed, however, either so much pus is produced as to make it impossible for this quantity to be discharged through the normal outlet passage, or the mucous membrane in the outlet passage has become so inspissated as a result of the inflammation that the outlet passage is completely occluded.

Now if it has been established by means of antroscopy that pus is contained in the maxillary antrum, the need is imposed of removing it. This can be done by vacuum suction drainage or washing. To this end, a more or less flexible tube is pushed up through the probe of the antroscope into the maxillary antrum.

It being impossible in the presence of an inflammation of the maxillary antrum to remove all of the inflammatory fluid in one session, and new inflammatory fluid furthermore being continuously produced for as long as the inflammation of the mucous membrane in the maxillary antrum has not been cured, repeated drainage of the maxillary antrum necessarily imposes itself. Repeated insertion of a drainage tube into the maxillary antrum by antroscopy or other instruments constitutes an unpleasant and in many cases painful operation for the

0137528

patient.

The present invention aims at providing an aid in the treatment of inflammations of the maxillary antrum, with the use of which the patient can be saved an appreciable part of the pain.

Accordingly, the invention provides a device for supplying a fluid substance to and draining it from the maxillary antrum, which device comprises a flexible tube as well as at least one arrester connected to one of the ends of the tube.

The physician in charge, having decided that undesirable substance is to be removed from the maxillary antrum and foreseeing that treatment will have to take place in several steps, will insert the device according to the invention into the cannula of the antroscopic troicart, after the probe has been removed from the antroscope. The tube part of the device according to the invention is passed through the nasal cavity into the maxillary antrum, where the device is held in position by means of the arrester or arresters. The device according to the invention having been introduced, the cannula is removed from the troicart, and the tube part of the device according to the invention is cut off to the proper size, so that the patient will carry with him, throughout the duration of the treatment, the tube which ensures communication between the maxillary antrum and the nasal cavity without being inconvenienced thereby, the tube being prevented by the arresters from becoming accidentally detached, such as by blowing the nose. During each visit to the physician in charge, the inflammatory fluid will be removed through the tube part of the device according to the invention, it also being possible in case of stubborn inflammatory residues to introduce a rinsing fluid through the device according to the invention into the maxillary antrum for the purpose of loosening the less fluid inflammatory residues. Thanks to the device according to the invention it is merely necessary during the first visit of the patient to perform the painful operation with the antroscopic troicart, the device according to the invention being capable of being removed in a simple manner upon completion of the treatment by pulling it out with the use of a slightly increased amount of force.

The device according to the invention, hereinafter called antroscopic drain, has the added advantage that it can be manufactured at low cost because of its beautiful simplicity, and that it can be sterilized in a simple manner.

It is preferable for the arrester or the arresters to be slewable between an arresting position and a free position, resistance being encountered during the swinging movement from the arresting position to the free position. As a result, the arresters, if no pressure is applied to them, will assume the arresting position and thus determine the position of the antroscopic drain. On the other hand, when the tube of the antroscopic drain is pulled with sufficient force, the arresters will swing up so as to assume the free position, thus allowing the end of the antroscopic drain to be removed without problems from the maxillary antrum. In inserting the antroscopic drain through the cannula of the antroscopic troicart, it is of course necessary to move the arresters upward so as to cause them to assume the free position, allowing the antroscopic drain to be pushed through the cannula.

For the purpose of simplicity of manufacturing the antroscopic drain according to the invention, it is desirable for the tube and the arresters to be manufactured of the same material and to merge into each other. On account of the resistance to deformation of the material of which the antroscopic drain is manufactured, the arresters, having first been pushed into the free position, swing back to the arresting position of their own accord. The halves of an end portion of the tube, having been severed lengthwise, can furthermore be deflected laterally so as to assume the arresting position, in which position they can be fixed by submitting them to a heat treatment.

In a preferred embodiment according to the invention, one of the ends of the tube merges into two semicylindrical arresters which lie opposite to each other. Such an embodiment of the device according to the invention can be manufactured in a particularly simple manner.

The dimensions of the antroscopic drain according to the invention are related to the intended purpose, and specifically to the dimensions of the antroscopic troicart. As has been mentioned in the above, the length of the tube is of secondary importance, since it is shortened to the required size after having been inserted. For practical reasons, a length of at least 20 cm or 2.5 times the length of the antroscopic troicart is considered desirable. The inside diameter of the tube should be large enough to allow the undesired fluid substance from the maxillary antrum to be properly passed through it, while the maximum outside diameter of the tube is determined by the inside diameter of the cannula of the troicart.

In this connection, the inside diameter of the tube in practice amounts to at least 1 mm, and the outside diameter of the tube to at most 4 mm. A preferred outside diameter of the tube is 2 to 3 mm in combination with a preferred inside diameter of 1.5 to 2 mm.

The device according to the invention can be manufactured of any material which is compatible with the surface of the body, causes no phenomena of irritation, can be sterilized in a simple manner, and can be easily worked up into a flexible tube. The antroscopic drain is preferably manufactured from a thermoplastic synthetic resin which is covered with a chemically inert, heat-resistant and nonadhesive coating of a fluorocarbon polymer, such as specifically polytetrafluoroethylene (Teflon ®).

For reasons of hygienics, the antroscopic drain according to the invention is sealed in an airtight envelope, sterilized in a usual manner, and only removed from the envelope briefly before being used by the physician in charge.

If desired, the free end of the tube of the antroscopic drain can be provided in advance with a sleeve, but this is not essential.

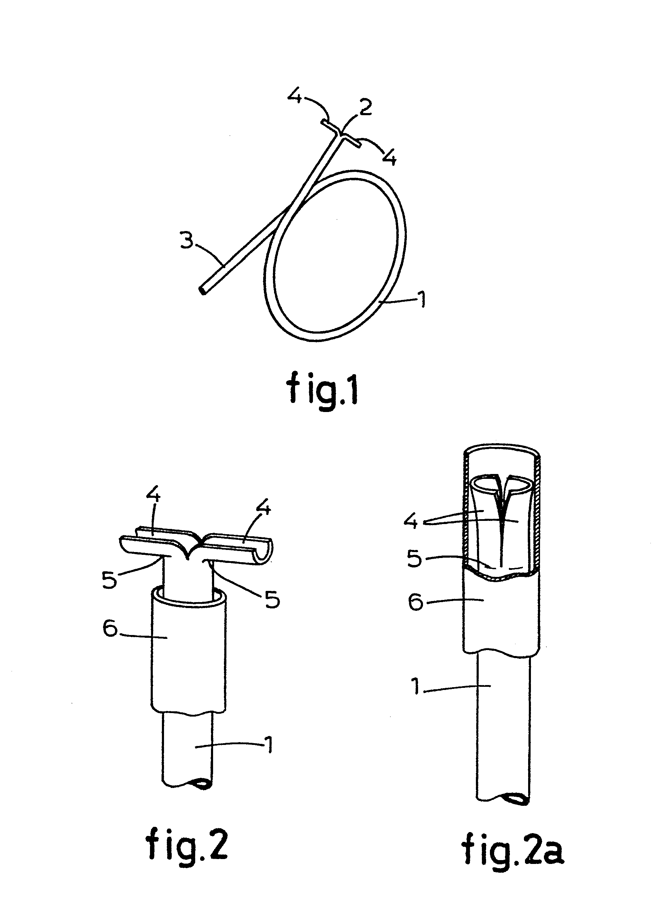

The invention is hereinafter explained in further detail on the basis of the drawing, where Fig. 1 represents an antroscopic drain according to the invention.

Fig. 2 shows in additional detail the top part of the antroscopic drain according to Fig. 1, with the arresters in the arresting position.

Fig. 2a corresponds to Fig. 2, except that the arresters are shown in the free position.

Fig. 3 shows details of another embodiment of the invention, with the arresters in the arresting position.

Fig. 3a corresponds to Fig. 3, except that the arresters are shown in the free position.

Fig. 4 is a sectional view of the face of a human skull, with the device according to the invention inserted in the maxillary antrum.

Fig. 1 shows an antroscopic drain according to the invention. It comprises a tube 1 with the one end 2 and the other end 3. Connected to the end 2 are two arresters 4 which, as shown, in no-load conditions assume an arresting position because they project beyond the circumference of the tube.

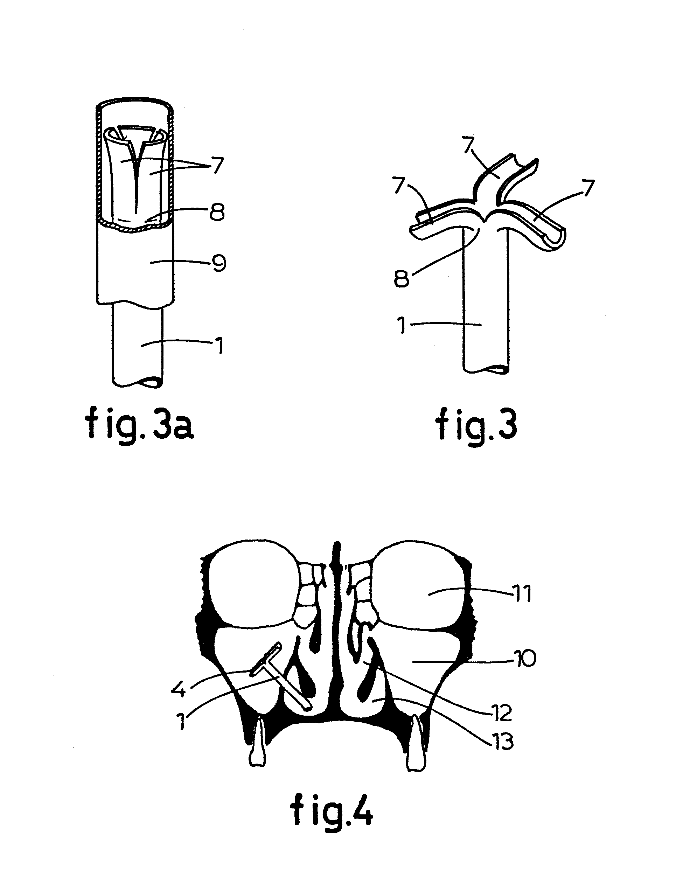

The portion of the antroscopic drain according to the invention near the one end 2 is shown in more detail in Figs. 2 and 2a. Fig. 2 shows the arresters 4 in the arresting position. The arresters 4, which are semicylindrical in this example of embodiment, are attached by a hinged joint 5 along the circumference of the end 2 to the tube 1. In no-load conditions the arresters 4 assume the position represented in Fig. 2, but since the antroscopic drain is made entirely of a flexible plastic, the arresters 4 can be moved upward or downward at the hinged joint 5. Under the influence of the resistance to deformation and of the elastic memory of the material of which the antroscopic drain if manufactured, the arresters 4, upon an applied load having been removed, will always resume the position represented in Fig. 2. Fig. 2a shows the arresters 4 in the free position, where they project co-axially with the surface of the tube 1, allowing the antroscopic drain to be moved freely through a duct, cannula or sleeve for insertion.

Also shown in Fig. 2a is the cannula 6 of an antroscope, which cannula 6 produces the pressure on the arresters 4 which causes them to be held in the free position.

When the antroscopic drain is inserted into the maxillary antrum, which situation is represented in Fig. 2a, the cannula 6 of an antroscope upon completion of the antroscopic examination still occurs in the passage from the nose to the maxillary antrum, which passage has been obtained with the aid of the antroscopic troicart. With the arresters closed, the antroscopic drain according to the invention is then inserted into the cannula 6 and advanced therein until the tip of the antroscopic drain has reached the end of the cannula 6 which is inserted in the maxillary antrum. During

0137528

this part of the operation, the antroscopic drain can be moved freely through the cannula 6, since the arresters 4 have been swung on the hinged joints 5 into the free position, thus causing negligible resistance at the inside surface of the cannula 6.

When the end 2 of the antroscopic drain is pushed beyond the end of the cannula, as shown in Fig. 2, the pressure at the arresters 4 is relieved, causing them to swing back to the arresting position, this being possible inside the space of the maxillary antrum. The cannula can then be removed, and the antroscopic drain according to the invention will remain behind for ensuring communication between the nasal cavity and the maxillary antrum. The arresters 4 prevent the antroscopic drain from being withdrawn from the maxillary antrum under a small tractive effort, but the antroscopic drain can be removed from the patient at the end of the treatment by pulling just slightly harder.

Fig. 3 and 3a show another example of embodiment of the antroscopic drain according to the invention, which can be manufactured highly efficiently and which meets all requirements. This embodiment likewise comprises a tube 1, and furthermore three furrow-shaped arresters 7, which are connected by hinged joints 8 to the end of the tube 1. Fig. 3 shows the arresters 7 in their arresting position, the free position being represented in Fig. 3a.

In the free position shown in Fig. 3a, the three gutter-shaped arresters 7 abut on each other while forming a cylindrical surface having the same diameter as the tube 1, allowing the antroscopic drain to be passed freely through a cannula. Fig. 3 shows the three furrow-shaped arresters 7 in the position where they are swung out, in such a way that the arresters 7 lie in a plane perpendicular to the tube 1, thus having assumed the arresting position.

Since some problems can be encountered in bringing the three arresters into the free position with the use of a sterile instrument, such as a small forceps, so as to allow them to be inserted into the cannula of the antroscopic troicart, the use of an auxiliary attachment can be desirable. To this end, an inserting sleeve 9, shown in Fig. 3a, is provided along with the antroscopic drain. The inserting sleeve 9, like the antroscopic drain, is made available in a sterile envelope, the arresters 7 being maintained in their free position inside the inserting sleeve 9. As a result, the inserting sleeve 9, and along with it the end 2 of the antroscopic drain, can be simply introduced in a sterile manner into the cannula 6 of an antroscopic troicart.

Fig. 4 is a sectional view of the front side of a skull. It is possible to perceive in it the maxillary antrum 10, the orbits 11, the central nasal ducts 12, and the inferior nasal ducts 13. The antroscopic drain 1 according to the invention extends through the passage from the inferior nasal duct 13 to the maxillary antrum, this passage being established with the aid of a troicart, and thus provides the desired communication between the maxillary antrum and the nose. The arresters 4, 7, having assumed the arresting position, prevent the antroscopic drain from being accidentally withdrawn from the maxillary antrum.

As a result of the hinged joints, 5, 8, having been submitted to heat treatment during the manufacturing process, the arresters 4, 7, when in the no-load state, assume the position shown in Fig. 2, whereas a small amount of pressure causes the two groups of arresters 4, 7 to be so closed as to assume the free positions represented in Figs. 2a, 3a.

- 8 -

0137528

## C L A I M S

1. Device for supplying a fluid substance into and draining it from the maxillary antrum, which device comprises a flexible tube as well as at least one arrester connected to one of the ends of the tube.

2. Device according to claim 1, characterized in that the arrester(s) is (are) slewable between an arresting position and a free position, resistance being encountered during the swinging movement from the arresting position to the free position.

3. Device according to claim 1 or 2, characterized in that one of the ends of the tube merges into two or more furrow-shaped arresters.

4. Device according to claims 1-3, characterized in that the inside diameter of the tube amounts to at least 1 mm, and the outside diameter of the tube to at most 4 mm.

5. Device according to claims 1-4, characterized in that it is manufactured from a thermoplastic synthetic resin which is coated with polytetrafluoroethylene.

6. Device according to claims 1-5, characterized in that it is packaged in sterile conditions in an airtight envelope.

7. Device according to claims 1-6, characterized in that it is packaged jointly with an inserting sleeve in an airtight envelope.

0137528

fig.1

fig.2

fig.2a

fig.3a

fig.3

fig.4

**European Patent Office**

**EUROPEAN SEARCH REPORT**

## DOCUMENTS CONSIDERED TO BE RELEVANT

EP 84201057.1

| Category | Citation of document with indication, where appropriate, of relevant passages | Relevant to claim | CLASSIFICATION OF THE APPLICATION (Int. Cl.4) |
|---|---|---|---|
| X | DE - A - 2 311 807 (EAST/WEST MED. PROD.) | 1-3,5 | A 61 M 25/00 |
| Y | * Fig. 4,5; page 4, first paragraph; page 5, second paragraph * | 6 | A 61 M 25/02 |
| A | | 4 | A 61 M 1/00 |
| | -- | | |
| X | US - A - 4 043 346 (D.F. MOBLEY et al.) | 1-5 | |
| Y | * Totality * | 6,7 | |
| | -- | | |
| Y | US - A - 4 230 115 (J.A. WALZ et al.) | 6,7 | |
| | * Abstract * | | |
| | -- | | |
| X | US - A - 3 592 197 (M.J. COHEN) | 1,2 | |
| A | * Totality * | 3,4,5 | TECHNICAL FIELDS SEARCHED (Int. Cl.4) |
| | -- | | |
| X | GB - A - 1 249 957 (W.M. KORTUM) | 1,2 | A 61 M 1/00 |
| A | * Totality * | 4,5,7 | A 61 M 25/00 |
| | ---- | | |

The present search report has been drawn up for all claims

| Place of search | Date of completion of the search | Examiner |
|---|---|---|
| VIENNA | 10-12-1984 | LUDWIG |